# EUROPEAN PATENT APPLICATION

(11) **EP 4 437 994 A1**
(43) Date of publication of application: **02.10.2024**
(21) Application number: 23165596.0
(22) Date of filing: 30.03.2023
(51) Int. Cl.: A61B 34/20, A61B 34/30, A61B 90/00

(54) **NAVIGATION OF ELONGATED ROBOTICALLY-DRIVEN DEVICES**

(71) Applicant: Koninklijke Philips N.V., 5656 AG Eindhoven (NL)
(72) Inventor: FOTOUHI, Javad, Eindhoven (NL); BALICKI, Marcin A., Eindhoven (NL); PATRICIU, Alexandru, 5656AG Eindhoven (NL); KYNE, Sean, Eindhoven (NL); VAN OORDE-GRAINGER, Shaun, Eindhoven (NL)
(74) Representative: Philips Intellectual Property & Standards

(57) **Abstract**

The present invention relates to navigation of elongated robotically-driven devices. In order to improve the workflow and to provide further assistance in handling an interventional device by a robot arrangement, a navigation system (10) for assisting in robotic anatomical pathway navigation of an elongated robotically-driven device is provided that comprises a data input (12), a data processor (14) and an output interface (16). The data input is configured to receive: robot-related data of a designated robot provided for performing at least one endoluminal-related task, device-related data related to inherent or mechanical properties of at least one elongated device to be driven by the designated robot, and subject-related anatomical pathway image data of a region of interest, in which the device is to be moved by the designated robot; and to provide the data to the data processor. The data processor is configured to compute an estimation of a working range for the at least one device in the anatomical pathway based on the robot-related data, the device-related data and the subject-related anatomical pathway image data being inputs for the estimation computation. The output interface is configured to provide the estimated working range.

## Description

### FIELD OF THE INVENTION

The present invention relates to navigation of elongated robotically-driven devices, and relates in particular to a navigation system for assisting in robotic anatomical pathway navigation of an elongated robotically-driven device, to a system for robotic anatomical pathway navigation and to a method for assisting in robotic anatomical pathway navigation of an elongated robotically-driven device.

### BACKGROUND OF THE INVENTION

Robotic solutions to treat endovascular diseases have been growing in the past few years. The applications of robotic-assisted procedures span multiple interventions, including coronary, peripheral, and neurovascular interventions. Various designs have been used for a robotic system, including an articulated arm or motorized robot module that can translate and rotate the vascular device. Depending on the intervention and the design of the robot, different devices with different working ranges and properties are required. For instance, robot-assisted neuro-endovascular procedures (stroke, aneurysm, etc.) will require a higher working range for devices to reach their targets in the carotids and brain. In robotic endovascular navigation, different endovascular devices are provided that can be exchanged. However, it has been shown that procedure interruptions for a device change can be cumbersome requiring finding new devices, unloading current ones, reloading new ones in a particular manner, and most importantly the exchanges significantly increase risk of complications, such as dissections and clots, as well as waste valuable time.

### SUMMARY OF THE INVENTION

There may thus be a need for improving the workflow and further assistance in handling an interventional device by a robot arrangement.

The object of the present invention is solved by the subject-matter of the independent claims; further embodiments are incorporated in the dependent claims. It should be noted that the following described aspects of the invention apply also for the navigation system for assisting in robotic anatomical pathway navigation of an elongated robotically-driven device, for the system for robotic anatomical pathway navigation and for the method for method for assisting in robotic anatomical pathway navigation of an elongated robotically-driven device.

According to the present invention, a navigation system for assisting in robotic anatomical pathway navigation of an elongated robotically-driven device is provided. The system comprises a data input, a data processor an output interface. The data input is configured or enabled to receive robot-related data of a designated robot provided for performing at least one endoluminal-related task and/or for driving an elongated-driven device. Such robot-related data may comprise data related to the driving of the elongated device by the robot. The data input is also configured or enabled to receive device-related data related to inherent and/or mechanical properties of at least one elongated device to be driven by the designated robot. The data input is further configured or enabled to receive subject-related anatomical pathway image data of a region of interest, in which the device is to be moved by the designated robot. The data input is configured to provide the data to the data processor. A non-transitory machine-readable storage medium or memory may be provided and encoded with instructions for execution by the data processor. The data processor is configured to compute an estimation of a working range for the at least one device in the anatomical pathway based on the robot-related data, the device-related data and the subject-related anatomical pathway image data (being inputs for the estimation computation). The output interface is configured to provide the estimated working range.

As an effect, providing information regarding the travel limits of a device used for the robot-assisted intervention allows an optimized use of the device. For example, if the working range is shown to be sufficient for a particular task provides confidence for the user that a sudden need for an exchange of the device is avoided. Or, if the working range is shown to be inadequate, the user can consider replanning or a change of the device to a preferred point in time, for example, before further navigating into the vascular structure. Unwanted procedure interruptions are either avoided or at least better integrated into the workflow. The provided knowledge about the (estimated) working range allows the user to smoothen the workflow. The knowledge about the working range thus supports a successful navigation.

In an example, the working range of a device dynamically changes as the user navigates. This means that, depending on the slack, kinks, device buckling, and energy buildups along the way, the maximum reach of the robotically-controlled device changes. Hence, a device that originally was anticipated to have sufficient length to reach a target, may half-way through navigation, appear to have insufficient length - suggesting to the user to either exchange the device early on, or clear the energy buildups from the system.

In an example, at least one repetition loop is provided that updates the estimated working range.

As an advantage, knowledge about the working range for i) the particular device, ii) the particular current anatomical situation and iii) the current robot arrangement assists the user in handling an interventional device(s) by a robot arrangement and thus improves the workflow.

In an example, the term robot arrangement relates to robot configuration and state.

According to an example, the data processor is configured to compute an estimation of a working range along different possible pathways for the at least one device. The output interface is configured to output the working range along the different possible pathways.

In an option, an assessment is computed for the different pathways based on predetermined weighting factor such as tortuosity of the path, number of branching-off passages, narrowing passages, width of the vessels, risk-importance of vessel passages and the like, such as slack and the amount of energy buildup which can be another input here. The determined assessment value is indicated to the user.

According to an example, the data input is configured to provide the device-related data comprising a plurality of device-related data for a plurality of different devices. The data processor is configured to compute a plurality of estimations for a working range based on the plurality of device-related data. The output interface is configured to output the working range along the different possible pathways.

In an option, an assessment is computed for the different devices based on predetermined weighting factor such as range of reach, steerability and the like. The determined assessment value is indicated to the user.

According to an example, the working range is provided as graphical information overlaid to anatomical image data of the region of interest. As an option, provided in addition or alternatively, the output interface is configured to provide the estimated working range as an image matrix where the maximum working range of each device within each pathway is marked uniquely in the image.

According to an example, the data input is configured to provide workflow data. The data processor is configured to compute the at least one estimation for a working range also based on the workflow data.

According to an example, a neural network-based controller is provided comprising convolutional filters that are configured to capture contextual patterns in the image data. As an option, provided in addition or alternatively, the data processor is configured to compute the estimation based on training of the neural network.

According to the present invention, also a system for robotic anatomical pathway navigation is provided. The system comprises a robotic arrangement and a navigation system according to one of the preceding examples. The robotic arrangement is configured for controlling and driving at least one device for insertion in and moving along an anatomical pathway of a region of interest of a subject. The navigation system is configured to provide an estimation of a working range of the at least one device within the anatomical pathway of the region of interest of the subject.

According to an example, an imaging arrangement is provided configured to provide the subject-related anatomical pathway image data. As an option, provided in addition or alternatively, the subject-related anatomical pathway image data is provided as 2D X-ray images.

This allows to use live images for estimating the working range, which on the one hand simplifies the procedure, as no further data is required regarding the present/current anatomical structure, and which on the other hand provides the most accurate information regarding the current anatomy in which the device needs to be navigated.

According to the present invention, also a method for assisting in robotic anatomical pathway navigation of an elongated robotically-driven device is provided. The method comprises the following steps:
- receiving robot-related data of a designated robot provided for performing at least one endoluminal-related task;
- receiving device-related data related to inherent or mechanical properties of at least one elongated device to be driven by the designated robot;
- receiving subject-related anatomical pathway image data of a region of interest, in which the device is to be moved by the designated robot;
- computing an estimation of a working range for the at least one device in the anatomical pathway based on the robot-related data, the device-related data and the subject-related anatomical pathway image data; and
- outputting the estimated working range.

According to an aspect, the travel limits of each device will depend on the relations between the current device, the robotic system, and the patient anatomy observed in the interventional image. It is provided to dynamically compute and display a robotically controlled endovascular device's maximum reach by combining imaging interpretation, device intelligence and robot-related data (which may include robot arrangement data such as for instance configuration of the robot (e.g. geometrical, articulation configuration, other design data, intrinsic parameters of the robot, robot state, robot settings, registration and/or calibration data...) further to data related to the driving such as for instance feedback data related to the driving (e.g. kinematics, encoder data from robotic motor(s), motion data - rotation, translation and/or rolling and/or positioning data...). The endovascular device's maximum reach can also be referred to as working range. The factors that determine the maximum reach of the device in the anatomy depend on the design of the robotic system, the placement of the endovascular device on the robot, the length of the devices, the access site location, e.g. radial or femoral, and the patient anatomy. The information regarding the working range can be presented as graphical overlays, textual, audio, or tactile feedback.

In an example, procedures may use multiple devices simultaneously, each has a working range and an optimal position to support the distal device inside it, if any.

The present invention focuses on combining new robotic technology with interventional imaging platforms. The working-range estimation system can be used together with e.g. fixed C-arm systems as well as mobile fluoroscopy systems. In an example, the working-range estimation system is designed to work with interventional guided therapy devices.

As an advantage, the usability and interface of image-guided robotic systems is enhanced. It can be made available as part of a robot core interface or within a software as a service solution.

According to an aspect, a working range of a device being driven by a robot device is determined based on a current anatomical situation. The process of computing the working range is based in particular on image data representing the current situation. The image data is taken and possible paths within the given anatomy are determined for a given device or selection of possible devices.

In further examples, multiple ways are provided for using image data. In an example, only the last image of the patient is used that captures the anatomy as well as the device within the vasculature at its latest position, e.g. if the device is already in the field of view.

In another approach, a sequence of images from past to present is used. In this case, for instance, the last 100 X-ray images from the same patient that show how the device has been moving so far in the vasculature is used during training/inference. These images can be from the same anatomical region, or they can be from various anatomical areas and different fields of views stitched together.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a navigation system for assisting in robotic anatomical pathway navigation of an elongated robotically-driven device.
Fig. 2 shows an example of a system for robotic anatomical pathway navigation.
Fig. 3 shows basic steps of an example of a method for assisting in robotic anatomical pathway navigation of an elongated robotically-driven device.
Fig. 4 shows another example of a schematic setup for assisting in robotic anatomical pathway navigation.
Fig. 5 shows a further example of a working scheme.
Fig. 6 shows an example of a presentation shown on a display.
Fig. 7 shows an example for an uncertainty indicator.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

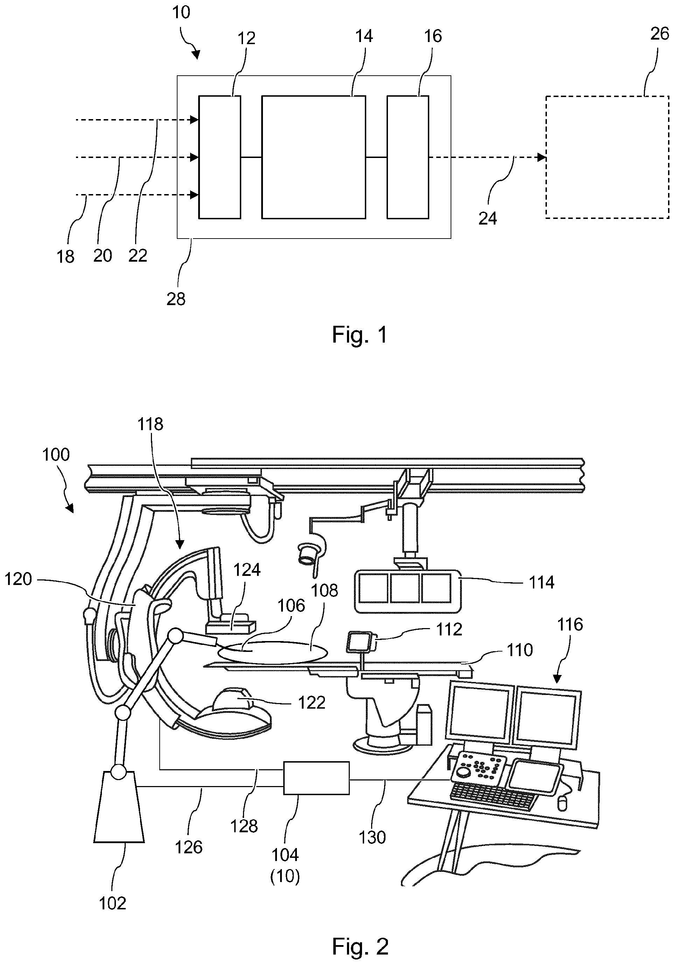

Fig. 1 schematically shows an example of a navigation system 10 for assisting in robotic anatomical pathway navigation of an elongated robotically-driven device. The system 10 comprises a data input 12, a data processor 14 and an output interface 16. The data input 12 is configured to receive robot-related data of a designated robot provided for performing at least one endoluminal-related task. The data input 12 is configured to also receive device-related data related to inherent or mechanical properties of at least one elongated device to be driven by the designated robot. The data input 12 is further configured to also receive subject-related anatomical pathway image data of a region of interest, in which the device is to be moved by the designated robot. The data input 12 is also configured to provide this data to the data processor 14. For the data processor 14, a memory is provided (not shown in detail) that stores instructions, and a processor that executes the instructions. The data processor 14 is configured to compute an estimation of a working range for the at least one device in the anatomical pathway based on the robot-related data, the device-related data and the subject-related anatomical pathway image data being inputs for the estimation computation. The output interface 16 is configured to provide the estimated working range.

Three arrows in broken lines indicate the input of the different types of data: A first arrow 18 indicates a supply or feed of the robot-related data. A second arrow 20 indicates a supply or feed of the device-related data. A third arrow 22 indicates a supply or feed of the subject-related anatomical pathway image data. A fourth arrow 22 in broken line indicates an output of data, i.e. providing the computed estimation of the working range. As an option, the estimation is shown on a display 26.

A frame 28 indicates that the data input 12, the data processor 14 and the output interface 16 are provided in a common housing in one option. In another option, the data input 12, the data processor 14 and the output interface 16 are provided as individual components.

In an example, not further shown in detail in the Figures, the working range comprises a at least one parameter of the group comprising: travel limit, working length and maximum reach.

Fig. 2 shows an example of a system 100 for robotic anatomical pathway navigation. It is noted that the system is shown in the context of an operating room with other equipment such as an imaging system as an option. The system 100 can also be provided as a stand-alone solution. The system 100 comprises a robotic arrangement 102 and an example 104 of the navigation system 10 according to one of the preceding examples. The robotic arrangement 102 is configured for controlling and driving, e.g. handling, at least one device 106 for insertion in and moving along an anatomical pathway of a region of interest of a subject 108. The navigation system 10 is configured to provide an estimation of a working range of the at least one device 106 within the anatomical pathway of the region of interest of the subject 108.

The robotic arrangement 102 is schematically indicated only. The robotic arrangement 102 can be a stand-alone robot with a stand and one or a plurality of manipulators. The robotic arrangement 102 can also be integrated into other movable equipment inside an operating room, e.g., the operating table. The robotic arrangement 102 can be supported on the floor, mounted to a wall structure or suspended from the ceiling. The robotic arrangement 102 can have one or several "arms" as manipulators. The manipulator can have a plurality of degrees of freedom of motion, such as three axis of freedom, or four, five or six degrees of freedom.

The robotic system can optionally include tracking systems such as those used in surgical navigation systems based on IR light or EM tracking or (optical) shape sensing technologies. Such tracking system can track the location of the robot arrangement relative to the patient and/or relative to the equipment in the operating room such as the x-ray system. The tracking system can provide information on the proximity of robot components to the patient access site and also the relative position of the robot components in the arrangement, in addition to the internal encoding methods (e.g. motor encoders, linear encoders, etc.) . This is one method to calibrate and or/track the relative displacement of the devices relative to the patient and the devices themselves. Alternatively, the tracking system can track the devices directly using EM markers, or optically tracked fiducials attached to the device(s), or using common computer vision methods with stereo cameras.

As an option, the subject 108 is shown arranged on a subject support 110. A beside controller 112 may be arranged in the vicinity. A suspended monitor arrangement 114 is also shown. Further, a console 116 for operating the equipment within the operating room is indicated. The console 116 can be arranged within the same room or in a separate room.

As an option, an imaging arrangement 118 is provided configured to provide the subject-related anatomical pathway image data. As an example, the imaging arrangement 118 is an X-ray imaging system with a movably mounted C-arm 120 with an X-ray source 122 and a detector 124 mounted to the opposing ends of the C-arm 120.

A first communication line 126 indicates a data-connection between the robotic arrangement 102 and the example 104 of the navigation system 10. A second communication line 128 indicates a data-connection between the imaging arrangement 118 and the example 104 of the navigation system 10. A third communication line 130 indicates a data-connection between the example 104 of the navigation system 10 and the console 116.

The data-connections can be provided as wire-based communication or as wireless communication.

In an option, the subject-related anatomical pathway image data is provided as 2D X-ray images.

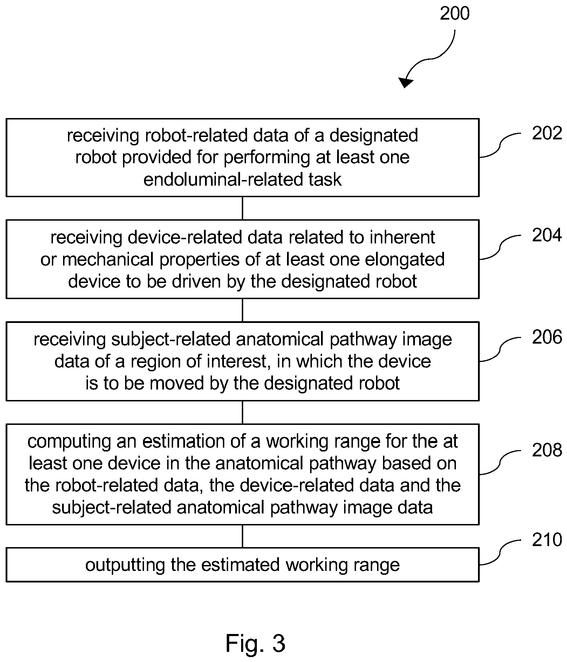

Fig. 3 shows basic steps of an example of a method 200 for assisting in robotic anatomical pathway navigation of an elongated robotically-driven device. The method 200 comprises the following steps: In a first sub-step 202, robot-related data of a designated robot provided for performing at least one endoluminal-related task is received. In a second sub-step 204, device-related data related to inherent or mechanical properties of at least one elongated device to be driven by the designated robot is received. In a third sub-step 206, subject-related anatomical pathway image data of a region of interest is received, in which region the device is to be moved by the designated robot. The three sub-steps can be provided simultaneously or subsequently in any order. In a further step 208, an estimation of a working range is computed for the at least one device in the anatomical pathway based on the robot-related data, the device-related data and the subject-related anatomical pathway image data. In a next step 210, the estimated working range is provided, e.g. to an operator.

Referring back to Fig. 1 and Fig. 2, the device for the endoluminal-related task, i.e. the device 106 for insertion in and moving along an anatomical pathway of a region of interest of a subject, may also be referred to as an interventional device.

In an example, the elongated robotically-driven device 106 is configured to be navigated in an anatomical structure. The anatomical structure relates to any type of structure of the subject which has differing properties for navigating the device. In an example, the anatomical structure relates to vessels or lumen, such as the vascular structure or organs.

The navigation system 10 for robotic anatomical pathway navigation can also be referred to as navigation device (for robotic anatomical pathway navigation) or as navigation arrangement (for robotic anatomical pathway navigation).

In a first option, the navigation system 10 is provided as a data processing arrangement. As an example, the navigation system is provided as a kit for upgrading existing systems. As another example, the navigation system is provided as a separate part of a system.

In a second option, the navigation system 10 also comprises the at least one device 106. As an example, the navigation system 10 is provided as a navigation set with a set of the devices 10, e.g. several different interventional devices.

In an example, when executed by the data processor 14, the instructions cause the system to receive at least one of the group of the robotic-related data, positioning or motion data, the device-related data and the image data.

The term "anatomical pathway navigation" refers to navigating, i.e. guided moving along a given anatomical lumen suitable for an insertion of a device like a guidewire, catheter or the like. The anatomical pathway or lumen can be any hollow anatomical structure such as a vascular structure, respiratory passages or the intestinal or gastrointestinal tract. In an example, the anatomical pathway or lumen is a naturally-occurring pathway.

The term "data input 12" refers to providing or supplying data for data processing steps. The data input 12 can also be referred to as image data input 12. The data input 12 can also be referred to as data supply, as image data supply, as image input, as input unit or simply as input. In an example, the data input 12 is data-connectable to an imaging source arrangement. The data input 12 is configured to receive the data, e.g. from respective data sources, and transfer same to the data processor 14.

The term "data processor 14" refers to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input and the output interface. In an option, the data processor 14 is configured to compute the range of the device when operated and moved by the robot. The data processor 14 can thus be referred to as "range compute unit". The range compute unit receives data collected before, during, or after robot-assisted surgical procedures and computes the possible working range for each device, e.g. in particular flexible device with respect to the patient, imaging, or robot reference frames.

The term "output interface 16" refers to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16. The output interface 16 is configured to receive the data from the data processor 14 and transfer same to e.g. a display.

The term "robot-related data" refers to data from the use of the robot. The term "robot-related data" refers to e.g. at least one of the group comprising encoding data, kinematics, end-effector poses, robot tracker data, user control input of the robot to move the robot. In an example, the robot-related data comprises robot-movement-related data, e.g. moving capabilities, options and restrictions. In another example, the robot-related data comprises robot-state-related data, e.g. the robot's status data. In another option, the robot-related data also comprises robot-calibration data. The robot-related data can also be referred to as robot data.

The term "device-related data" refers to data of the mechanical features of the device, such as overall length, working length, width, x-ray opacity, stiffness, shape, articulation model (for steerable devices), the way it is mounted to the robot and the like. The device-related data comprises data related to the device. As an example, the device-related data comprises inherent properties of the device and parameters of the interface with the robot. The device-related data can also be referred to as device data. The working length may be the section length that can go inside another device.

The term "subject-related anatomical pathway image data" refers to image data of the navigation from the imaging system. In an example, the anatomical pathway image data is provided as vascular structure image data. The subject-related anatomical pathway image data can also be referred to as subject anatomical pathway image data or subject pathway image data.

The term "endoluminal-related task" refers to tasks within the lumen, such as within blood vessels, the respiratory system or other hollow sections within the anatomical structure. The term "endoluminal-related task" can also be referred to as "endovascular-related task".

The term "working range" refers to at least one of the group comprising travel limit, working length and maximum reach.

The imaging data can be two-dimensional (2D) or three-dimensional (3D). Preferably, 2D image data is provided, such as live fluoroscopy images.

In an option, the anatomical pathway navigation refers to endovascular navigation; and the device-related data is data relating to an endovascular device.

In an example, the output interface 16 is configured to provide the estimated working range for assisting an operator.

In an example, the data processor 14 is configured to compute an estimation of a working range for the at least one device in the anatomical pathway in relation to the target data based on the robot-related data.

According to the invention, the "estimation", or better its corresponding model, is based on e.g. multiple movement probabilities of an elongated device type having kinematics in constraints of an anatomical environment.

In an example, the model is obtained by training a neural network using retrospective robotic and imaging data. The neural network can be designed with multiple channels at the output layer, where each channel corresponds to a separate possible estimation of the device limit for a different movement probability.

The term "travel limit" relates to a travel distance, e.g. a distance along a certain travelled path, of the device when moved by the robot.

The term "working length" relates to a length of a vessel section(s), inside which the interventional device is moved, along which, length a working of the interventional device is possible. The working length may thus be a part of the travel distance. In an example, working length is the amount that the device (tip) can reach in the vasculature given the constraints.

The term "maximum reach" relates to a possible maximum distance to which the device can reach out. In an example, the maximum reach is the actual position in the vessel. The maximum reach may be a distance in which a working function is still possible. In another option, the maximum reach may be longer than the working length.

In an example, not further shown in detail in the Figures, the data processor 14 is configured to compute an estimation of a working range along different possible pathways for the at least one device. Further, the output interface 16 is configured to output the working range along the different possible pathways.

In an example, not further shown in detail in the Figures, the data input 12 is configured to provide the device-related data comprising a plurality of device-related data for a plurality of different devices. The data processor 14 is configured to compute a plurality of estimations for a working range based on the plurality of device-related data. Further, the output interface 16 is configured to output the working range along the different possible pathways.

In an option, estimations of a working range along different possible pathways are provided for a plurality of different devices.

As an example, the maximum working range of each device within each vessel, or other anatomical pathway, is marked uniquely in a displayed image. An example output of the system is an image matrix where the maximum working range of each device within each vessel is marked uniquely in the image. For instance, the coordinate corresponding to the maximum reach is marked with a different intensity compared to other pixels; or the entire trajectory in each vessel up to the coordinate of the maximum reach is marked with a different intensity.

As an effect, the visualization of device limits allows the user, e.g. interventionalists, to observe, in real-time, the working range that each robotically controlled device can reach with current settings and imaging feedback, hence, adjusting their catheterization strategy and devices timely and accordingly. As another exemplary effect, the visualization of device limits allows the user to adjust the relative positioning of the robot if needed. The term "relative" relates to the position in relation to the subject. As an option, additionally or alternatively, the relative positioning of the subject can be adjusted if needed.

In an option, the processor is configured to compare the working ranges and to provide a ranked proposal of at least two working ranges.

In an example, the device-related data comprises a plurality of device-related data for a plurality of different devices.

In an example, not further shown in detail in the Figures, the working range is provided as graphical information overlaid to anatomical image data of the region of interest. In an option, provided in addition or alternatively, the output interface is configured to provide the estimated working range as an image matrix where the maximum working range of each device within each pathway is marked uniquely in the image.

As an example, the graphical information is provided as an indicator for the travel limit, working length or maximum reach of the catheter.

In an example, the travel limit refers to the possible path-length the device 106 can move along, i.e. travel.

In another example, the working length refers to the possible path-length the device 106 can travel while still being capable of providing a designated operating task. Such a task can be a forward motion, e.g. an imaging or ablation procedure, a backward motion procedure, such as pullback, or even a repetitive back and forth working procedure.

In an example, the maximum reach refers to the possible length along the vessel which still allows the proper operating procedure, such as the distal imaging reach of an imaging catheter.

For example, the coordinate corresponding to the maximum reach is marked with a different intensity compared to other pixels. In another example, the entire trajectory in each pathway segment, e.g. vessel, up to the coordinate of the maximum reach is marked with a different intensity.

In an example, not further shown in detail in the Figures, the robot-related data comprises at least one of the group of: data obtained from the robot such as the robot encoder information, robot travel limits, CAD designs, forward kinematics, inverse kinematics, velocities, accelerations, end-effector poses, user controller input, and robot placement with respect to the patient.

In an example, not further shown in detail in the Figures, the device-related data comprises at least one of the group of: device-specific insights, device type, device length, device stiffness, data about device-robot relations such as the distance between the tip or end of the device from the robot actuation units, rollers or grippers, steerability information and manufacturer.

In another example, the device-related data relates to robot data; as an example, rollers and grippers may be robotically-driven elements of the robot, as well-known in the art, to roll or to grip, which may contact the elongated device and may directly actuate a movement to the elongated device, such as a translation or rolling. A robot may be further arranged, apart from rollers and grippers, to rotate, translate and/or roll the elongated device.

Hence, "rollers" and "grippers" are actuators, related to robot data. As an example, calibration is supplied to provide relevant robot data related to the actuators. Calibration data provides knowledge as to the relative positions of the devices in a device stack, such as 0 position + length + robot position.

In an example, the steerability information relates to specific devices that are able to articulate. As an option, this is provided be under the umbrella of the device model which includes shape behavior and the controllability; specific articulation for steerable devices is added to standard translaterotate control.

In an example, not further shown in detail in the Figures, the subject-related vascular structure image data comprises at least one of the group of: data obtained from medical imaging, such as fluoroscopy or ultrasound imaging, and pre- or intra-operative medical imaging data, such as 3D rotational angiographies, CT, CBCT, and MRI images. Further, the data processor 14 is configured to determine anatomical structures in the image data to identify anatomical pathways suitable for navigation of the at least one device within the anatomical pathways. As an option, provided in addition or alternatively, the subject-related anatomical pathway image data comprises target data comprising at least one of the group comprising a target and a path segment.

As an option, the subject-related anatomical pathway image data of a region of interest comprises at least one of the group of vascular structure, respiratory passages or intestinal passages.

In an option, the image data is provided as 2D image data, e.g. live or current fluoroscopy image data.

The data processor is configured to provide a segmentation for identifying the anatomical pathways.

In an example, not further shown in detail in the Figures, the data input 12 is configured to provide workflow data. Further, the data processor 14 is configured to compute the at least one estimation for a working range also based on the workflow data.

In an example, the workflow data comprises information related to an access site. As an example, depending on the access point (femoral or radial), the distance between the device starting point (device base) to the target anatomy changes, hence it directly affects the working length.

In an example, the workflow data comprises information related to a target anatomy. As an example, information from the target anatomy can determine the tortuosity along the way, which directly affects the estimation of the working length.

In an example, the workflow data comprises information related to a type of procedure being applied. As an example, data regarding the procedure type, e.g. aneurysm coiling in brain, embolization for stroke, etc., implicitly informs the system regarding some of the complications with regards to device maneuvering, navigation path, and the like. As an option, when applying a neural network, the neural network learns from the input data.

In an example, the workflow data comprises information about kinks/buckling/slack or the like. As an example, these components will reduce the effective working length of the device. For instance, when the system is aware of a kink or high slack in the system, it can adjust its prediction to output a shorter working length.

In an option, the above input is placed in a descriptor vector, digitized afterwards, and fed to a neural network both during training and inference.

As an example, the access site comprises (right/left) femoral and/or radial access points. As an example, the procedure type comprises mechanical thrombectomy or coiling.

As an advantage, e.g. in robotic endovascular interventions, a user's correct choice of devices already considering their maximum reach inside the anatomy is facilitated. As an option, a robot is placed near the access area and multiple candidate devices are selected, e.g. catheters, guidewires, guide catheters, microwires and the like, for possible use, i.e. deployment during the intervention. By determining, i.e. estimating the working range, candidates of the device with a length not appropriate for the particular task can be deselected, which avoids a situation in which a user would have to replace a chosen device due to lack of working range. Thus, by providing an estimation of the working range, a procedure's efficiency is significantly supported and enhanced.

Estimating working ranges also allows the use of standard endovascular devices for robotic navigation, where the standard devices may primarily be designed for manual navigation. Such devices may have a length that is not sufficient for certain long range robotic-assisted procedures, but might match in a plurality of robotic-assisted procedures. The estimating of the working ranges also addresses robots which with limited travel ranges, which directly impacts the working length of the robotically controlled device.

By facilitating to choose devices with the correct lengths, the need for manual steps like multiple device exchanges or robot displacement to gain additional moving range, which would interrupt the procedure and which would require significant staff engagement and consequently prolongs the procedure, is avoided or at least minimized.

The estimation can be implemented in a robot assisted system for conducting the respective task. When providing image data that reflects the current anatomical situation, the user is provided with real-time information regarding the maximum working range of each device within the anatomy. The estimation may consider a plurality of parameters, such as the device shape, slack, anatomy, robot type and device type. As an effect, the medical workflow is improved in a beneficial way and a new level of confidence is provided to e.g. the interventionalists.

The assisting device is designed to use some or all the above data and estimate an output that encodes the range of each device. The output of the device can be of numerical, tabular, image, or other formats.

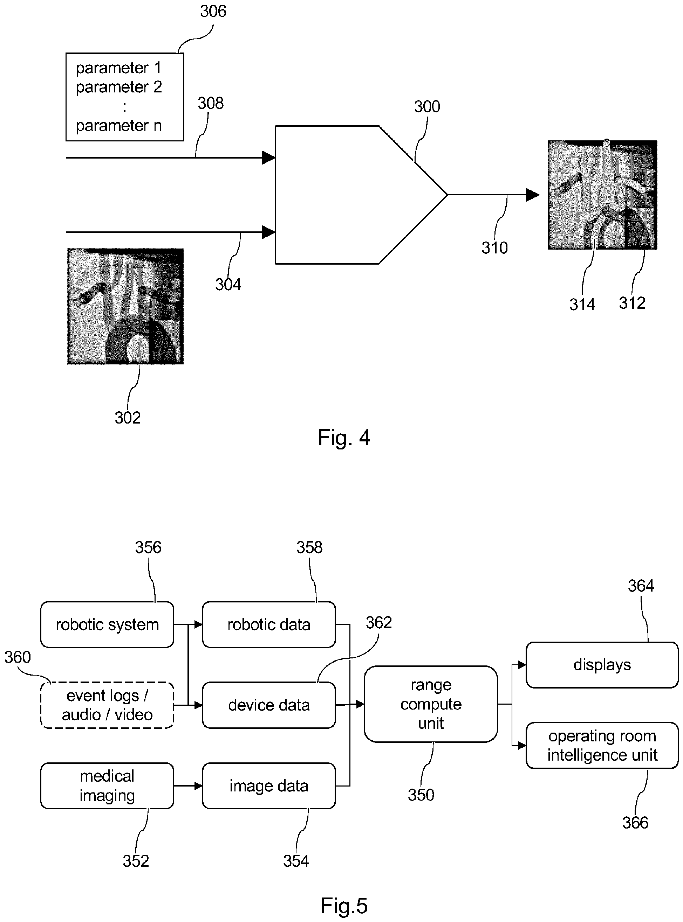

Fig. 4 shows a schematic setup of another example. A range estimator 300 (in the center portion) is connected to data supply (on the left). A 2D X-ray image 302, as an example for live or current image data, representing an anatomical image of a region of interest of a subject, is indicated as an input. A first arrow 304 indicates the supply or input of the data to the range estimator 300. Further, a plurality of parameters 306 is provided and supplied as further input, indicated by second arrow 308, to the range estimator 300. The parameters relate to both the device and the robot used for operating the device. Examples for the parameters are device type, device entry point, especial devices, device mount on the robot and robot encoder parameters. The range estimator 300 computes the working range of the device for the given anatomy, and provides this as an output, indicated by a third arrow 310. An illustration 312, as an output, shows the anatomy, e.g. as an X-ray image, such as an angiography image, overlaid by a graphical representation 314 of the working range. Fig. 4 shows the working range estimator module using two sets of input data: first, robot/device state, which includes the intrinsic parameters of the robot and the device and their relative relations; and second, the imaging feedback showing the current configuration of the device inside the vasculature. The output of the "range estimator" module 300 is then overlaid as the maximum working range with respect to the interventional images.

Fig. 5 shows a further example of a working scheme. A range compute unit 350 is data-connected to a medical imaging 352, i.e. an imaging device, supplying image data 354 to the range compute unit 350. Further, the range compute unit 350 is data-connected to a robotic system 356 supplying robotic data 354 to the range compute unit 350. Still further, as an option, a workflow source 360 providing e.g. event logs, or audio data of the current scenery in an operating room, or video of user activities in the operating room are provided and supplied to the robotic data, as well as provided as device data 358 to the range compute unit 350. The robotic system 356 may also supply data to the device data 362.

The range compute unit 350 is data-connected to one or several display 364. As an option, the range compute unit 350 is data-connected to an operating room intelligence unit 366 making further use of the generated working range data.

In a particular embodiment, the range compute unit 350 or range estimator 300 or controller or resulting model has been developed based on parameters defined from movement probabilities of elongated device types (types being defined in device data) having potential kinematics (included in robot data) in determined anatomical environment (e.g. vascular or respiratory or other endoluminal structure included in anatomical pathway image data), and may involve parameters related to target region or location (may be included in anatomical pathway image data).

These parameters may be entered manually or generated based on sets of data.

In a more particular embodiment, the range compute unit 350 or range estimator 300 or controller or resulting model has been trained based on sets of previous data, which may include robot data, device data, anatomical pathway image data and may also include determined limitations of working range (see more exemplary details in subsequent sections).

In an example, not further shown in detail in the Figures, the range compute unit 350 or range estimator 300 or controller or resulting model comprises a neural network-based controller comprising convolutional filters. Filters may be configured to capture contextual patterns in the image data. As an option, provided in addition or alternatively, the data processor is configured to compute the estimation based on training of the neural network.

In an example, contextual patterns are learned as weights of the convolutional kernels and are extracted as feature maps from the input data. For instance, with supervised training, the weights are estimated based on minimizing the distance between the estimated working range and the ground-truth working range labels. Example of low-level contextual features include landmarks on the device and anatomy and device boundaries in the image. High level contextual patterns include the overall structure of the device with respect to vessel structure.

In a further example, the contextual patterns are spatial contextual patterns. In an example, spatial contextual patterns are the ones that capture 2D or 3D spatial relations between different anatomies and devices based on the input data. These can be low-level patterns, such as the position of different edges in the images, or can be high level patterns, such as the overall position of device in the vasculature, or the registration between the robotic data/state and the device location in the 3D vasculature. Convolutional kernels are used in the neural networks to capture spatial context as described above.

In another example, the contextual patterns are non-spatial contextual patterns.

In an example, the neural network also uses fully connected layers to capture vectorized and numerical patterns and embeddings from robot data, device-related data or workflow data. The neural network may also use recurrent layers, such as RNNs, LSTMs, transformers etc., to capture temporal dependencies when using time-series data.

In an option, for training purposes of the network-based controller, the various input data is provided as synthetic data obtained in a computer simulation environment including use of different robotic settings, synthetic data from device models and target anatomies, as further explained in subsequent sections of this disclosure.

In an option, a range estimator training phase is provided. The weights used in the neural network for range estimation are learned and stored during the training phase. To create training data, various data are collected from the manual navigation of the robot. All relevant data, such as imaging, robot, device, workflow, data etc., are stored during the data acquisition step. Every time the robot reaches a limit during the manual navigation, the image coordinates and the corresponding interventional data (image, robot, device, workflow data) are stored. The coordinates of the device limit are then used as ground-truth labels. Finally, the training data and the corresponding labels are used for training the neural network.

During training, the neural network weights are optimized using a back-propagation process. As an example, in every iteration, the neural network predictions are compared against the ground-truth labels using a distance function. Some relevant distance functions for training the neural network may include (but are not limited to) L-2 distance (Euclidean), L-1 distance, binary cross-entropy, dice loss.

As a further option, a range estimator inference phase is provided. During the inference phase, the weights obtained during training are stored and used to compute the expected maximum working range. In this step, real-time interventional data (image, robot, device, workflow, etc.) are fed to the neural network controller in the same format used during the training. Finally, a forward-pass of input data through the neural network model yields the output.

As a still further option, learning in simulation is provided. For example, in a computer simulation environment, simulation interventional sessions are generated from different settings of the endoluminal (e.g. endovascular) robot, different endoluminal device models, and target anatomies. Each device is synthetically advanced through all branches relevant to the target procedure, and device limits are obtained and stored with every new setting. The synthetically generated [robot, device, imaging] data are then used as input signals to the controller that was introduced in the main claim. The device limits will be used as ground-truth labels corresponding to the input data. The set of input and ground-truth labels generated here will be used for training the neural network controller introduced above.

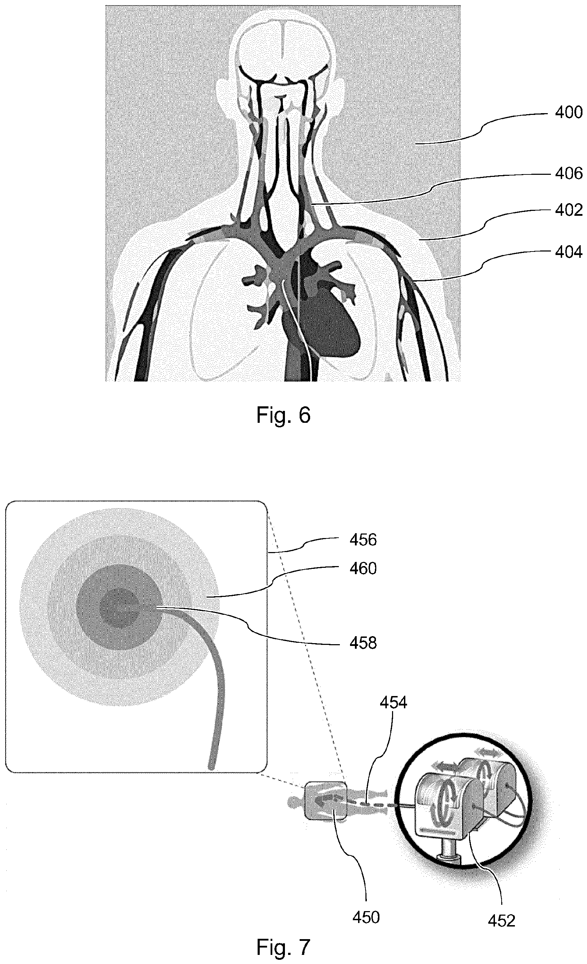

In an option, assignment of graphical elements is provided. As an example, it is provided to assign or change a single or multiple visual, audio, or textual elements on a graphical display based on the operating range of the robotically controlled device. An example of this embodiment is augmenting the travel range on the fluoro, contrast, or roadmap images, as shown in Fig. 6.

In Fig. 6, an example of a presentation shown on a display is provided. An image 400 represents a region of interest of a subject 402 showing a respective illustration indicating a vascular structure 404. An overlaid indicator 406, e.g. highlighting, indicates the computed working range. Fig. 6 shows the working range overlaid on the display interface. The overlay can be augmented onto 2D or 3D acquisition (or simulated) images. As an option, the images are fluoro, DRRs, DSA, roadmap, CBCT, CT, etc.

In an example, not further shown in detail in the Figures, the data processor 14 is configured to determine a working range uncertainty. The output interface 16 is configured to provide an indication of the working range uncertainty.

Fig. 7 shows an example for a probability or uncertainty indicator. In the lower right part, a subject 450 is depicted in a simplified manner. Further, a robotic drive 452 driving a device 454 partly inserted in the subject 452 is shown. In a magnified portion 456, a distal and 458 of the device 454 is shown. A plurality of circles 460 in different grey values, or colors, or patterns, indicate different degrees of certainty, i.e. an uncertainty indicator for the computed range of the (distal tip of the) device 454.

As an option, device range uncertainties are estimated using Monte Carlo's dropout that approximates Bayesian inference for a deep Gaussian process. To compute uncertainty, a subset of the neurons in the neural network controller presented in the primary embodiment is switched off during the forward pass to trigger the dropout. Next, every incoming data batch is passed through the model multiple times, e.g. ten times. Every time, the dropout mechanism results in a slightly different form of the model that can subsequently result in a different working range estimation by the neural network. All these processes' outcomes are aggregated to compute the upper and lower bounds for the device's working range. Finally, these uncertainty bounds are visualized on display. An example form of this visualization may be using different colors or dotted lines for the uncertainty bounds overlaid on the display image.

In a first example, the system is provided comprising the robotic arrangement and the navigation system.

In a second example, the system is provided comprising the robotic arrangement and the navigation system as well as the at least one device. The interventional device is configured for insertion into a pathway of a subject, for example a vascular structure.

In an option, the system is provided comprising several devices, i.e. at least two or more. For example, a set of devices is provided.

In an example, the robotic arrangement is configured to be directly registered to the subject for computing estimation of the working range of the robotically controlled and driven or handled device.

In an option, a device working range is estimated based on registration. The robotic system is configured to be directly registered to patient anatomy to compute the operating range of a robotically controlled device. This registration loop is closed by finding the relation between the robotic system and the anatomy visualized in the X-ray system. One approach to compute the registration transformations via pre- or intra-operative 3D imaging and 2D/3D registration techniques. In an option, a user feedback is provided once a predetermined amount of the working range has been reached. The user feedback is provided as at least one of the group comprising a visible feedback, audible feedback or tactile feedback.

For example, the device assigns tactile feedback such as vibration to a physical controller when the device is expected to reach the limits computed in the prior embodiments.

In an example, the predetermined amount is provided as half of the working range, e.g. approximately 50%, or as less than half of the working range such as 75%, 80%, 85%, 90% or 95% or even more. In another example, the predetermined amount is provided as the complete working range. As an example, the visible feedback is provided as an overlay to the anatomical image, or as a separate light signal. In an additional or alternative option, a vibration or other tactile feedback is provided to the user when the travel limit is reached or is about to be reached, such as via an operating handle for the user's feet or hand or as a vibration of a floor part.

In an option, a system control is changed based on working range. For example, the robot's controller changes the gain or speed of the system based on the distance of the device tip from the working limits of the device computed with the methods described in the prior embodiments.

In an option, a system control change comprises a change in the imaging system, e.g. frame-rate changing, resolution adaption, magnifying or the like, if near the limit of the working range.

The term "subject" may also be referred to as individual. The "subject" may further also be referred to as patient, although it is noted that this term does not indicate whether any illness or disease is actually present with the subject.

In an example, a computer program is provided that comprises instructions which, when the program is executed by a computer, cause the computer to carry out the method of the preceding examples.

In an example, a computer program or program element for controlling an apparatus according to one of the examples above is provided, which program or program element, when being executed by a processing unit, is adapted to perform the method steps of one of the method examples above. In an option, a computer readable medium having stored the computer program of the preceding example is provided.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A navigation system (10) for assisting in robotic anatomical pathway navigation of an elongated robotically-driven device, the system comprising:
- a data input (12);
- a data processor (14); and
- an output interface (16);
wherein the data input is enabled: to receive: robot-related data of a designated robot provided for driving the elongated-driven device in order to perform at least one endoluminal-related task, such robot-related data including robot arrangement data and/or data related to said driving; device-related data related to inherent or mechanical properties of at least one elongated device to be driven by the designated robot; and subject-related anatomical pathway image data of a region of interest, in which the device is to be moved by the designated robot; and to provide the data to the data processor;
wherein the data processor is configured to compute an estimation of a working range for the at least one elongated device in the anatomical pathway based on the robot-related data, the device-related data and the subject-related anatomical pathway image data being inputs for the estimation computation; and
wherein the output interface is configured to provide the estimated working range.

2. System according to claim 1, wherein the working range comprises a at least one parameter of the group comprising: travel limit, working length and maximum reach.

3. System according to claim 1 or 2, wherein the data processor is configured to compute an estimation of a working range along different possible pathways for the at least one device; and
wherein the output interface is configured to output the working range along the different possible pathways.

4. System according to claim 1, 2 or 3, wherein the data input is configured to provide the device-related data comprising a plurality of device-related data for a plurality of different devices;
wherein the data processor is configured to compute a plurality of estimations for a working range based on the plurality of device-related data; and
wherein the output interface is configured to output the working range along the different possible pathways.

5. System according to one of the preceding claims, wherein the working range is provided as graphical information overlaid to anatomical image data of the region of interest; and
wherein the output interface is configured to provide the estimated working range as an image matrix where the maximum working range of each device within each pathway is marked uniquely in the image.

6. System according to one of the preceding claims, wherein the robot-related data comprises at least one of the group of: data obtained from the robot such as the robot encoder information, robot travel limits, CAD designs, forward kinematics, inverse kinematics, velocities, accelerations, end-effector poses, user controller input, and robot placement with respect to the patient.

7. System according to one of the preceding claims, wherein the device-related data comprises at least one of the group of: device-specific insights, device type, device length, device working length, device stiffnesses, device shape, data about device-robot relations such as the distance between the tip or end of the device from the robot actuation units, force, torque, velocities, robot type, device manipulation mechanism (rollers, grippers, belts, fixed) steerability information and manufacturer.

8. System according to one of the preceding claims, wherein the subject-related endoluminal structure image data comprises at least one of the group of: data obtained from medical imaging, such as fluoroscopy or ultrasound imaging, and pre- or intra-operative medical imaging data, such as 3D rotational angiographies, CT, CBCT, and MRI images; and
wherein the data processor is configured to determine anatomical structures in the image data to identify anatomical pathways suitable for navigation of the at least one device within the anatomical pathways; and
wherein the subject-related anatomical pathway image data comprises target data comprising at least one of the group comprising a target and a path segment.

9. System according to one of the preceding claims, wherein the data input is configured to provide workflow data; and
wherein the data processor is configured to compute the at least one estimation for a working range also based on the workflow data.

10. System according to one of the preceding claims, wherein a neural network-based controller is provided comprising convolutional filters that are configured to capture contextual patterns in the image data; and
wherein the data processor is configured to compute the estimation based on training of the neural network.

11. System according to one of the preceding claims, wherein the to the computed estimation comprises determination of a working range uncertainty; and
wherein the output interface is configured to provide an indication of the working range uncertainty.

12. System (100) according to any of the previous claims, further comprising:
- a robotic arrangement (102); and
- a navigation system (10) according to one of the preceding claims;
wherein the robotic arrangement is configured for controlling and driving the navigation of at least one elongated device along an anatomical pathway of at least one region of interest of a subject, the robotic arrangement (102) being further arranged to provide robot-related data;
wherein the navigation system is configured to provide an estimation of a working range of the at least one elongated device within said anatomical pathway of the region of interest of the subject.

13. System according to claim 12, wherein an imaging arrangement (118) is provided configured to provide the subject-related anatomical pathway image data; and
wherein, preferably, the subject-related anatomical pathway image data is provided as 2D X-ray images.

14. A controller arranged to provide an assistance to the drive of an elongated robotically-driven device in robotic anatomical pathway navigation, comprising:
a data input (12);
a data processor (14); and
an output interface (16);
wherein the data input is enabled: to receive: robot-related data of a designated robot provided for driving the elongated-driven device in order to perform at least one endoluminal-related task, such robot-related data including robot arrangement data and/or data related to said driving; device-related data related to inherent or mechanical properties of at least one elongated device to be driven by the designated robot; and subject-related anatomical pathway image data of a region of interest, in which the device is to be moved by the designated robot; and to provide the data to the data processor;
wherein the data processor is configured with computer program instructions to compute an estimation of a working range for the at least one elongated device in the anatomical pathway based on the robot-related data, the device-related data and the subject-related anatomical pathway image data being inputs for the estimation computation; and
wherein the data processor is further configured to output via the output interface the estimated working range.

15. Computer program comprising instructions stored and encoded in a non-transitory processor-readable medium, which, when the program is executed by the controller as claimed in claim 14.
